(19)

Europäisches
Patentamt

European
Patent Office

Office européen
des brevets

(11)     **EP 3 752 052 B1**

(12)                          **EUROPEAN PATENT SPECIFICATION**

(45) Date of publication and mention
of the grant of the patent:
**06.10.2021   Bulletin 2021/40**

(21) Application number: **19704323.5**

(22) Date of filing: **11.02.2019**

(51) Int Cl.:
*A61B 5/021* *(2006.01)*      *A61B 5/022* *(2006.01)*

(86) International application number:
**PCT/EP2019/053257**

(87) International publication number:
**WO 2019/155048 (15.08.2019 Gazette 2019/33)**

(54) **CONTROLLING A WEARABLE CUFF**

STEUERUNG EINER WEARABLE-MANSCHETTE

COMMANDE D'UN BRASSARD VESTIMENTAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.02.2018   EP 18156346**

(43) Date of publication of application:
**23.12.2020   Bulletin 2020/52**

(73) Proprietor: **Koninklijke Philips N.V.
5656 AG Eindhoven (NL)**

(72) Inventors:
• **AELEN, Paul**
**5656 AE Eindhoven (NL)**
• **KUENEN, Maarten, Petrus, Joseph**
**5656 AE Eindhoven (NL)**

(74) Representative: **Philips Intellectual Property &
Standards
High Tech Campus 5
5656 AE Eindhoven (NL)**

(56) References cited:
**WO-A1-2017/129495      US-A1- 2012 220 884
US-A1- 2014 257 116      US-A1- 2017 245 769**

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

**Description**

FIELD OF THE INVENTION

The idea relates to an apparatus and method for controlling a wearable cuff for use in measuring blood pressure.

BACKGROUND OF THE INVENTION

Blood pressure (BP) or, more precisely, arterial blood pressure, is the pressure exerted by circulating blood on the arterial vessel walls. It is one of the key vital signs to establish patient well-being and therefore needs to be monitored for patients at risk. Blood pressure is a periodic signal, which rises at each contraction of the heart and decreases in between heart beats. It is typically described by systolic blood pressure (SBP), diastolic blood pressure (DBP) and mean arterial blood pressure (MAP), where systolic blood pressure is the maximum blood pressure during heart cycle, diastolic blood pressure is the minimum blood pressure during heart cycle, and mean arterial blood pressure is the average blood pressure during a heart cycle.

Different techniques exist by which blood pressure can be determined and these can be classified as invasive or non-invasive measurement techniques. Typically, non-invasive measurement techniques are cuff-based, which require an inflatable cuff to be placed around a limb (which is usually the upper arm) of a subject. The pressure in the cuff is then changed to infer blood pressure. There are two common methods that use a cuff in this way, which are referred to in the art as the auscultatory method and the oscillometric method respectively.

The auscultatory method for blood pressure measurement is based on the appearance and disappearance of sounds created by the artery under the cuff during the period that the cuff pressure is changed. These sounds are referred to in the art as Korotkoff sounds. The pressures at which the Korotkoff sounds appear and vanish are indicative of DBP and SBP with Korotkoff sounds appearing at each heart beat between DBP and SBP. The measurement of sound can be performed manually with a stethoscope that is placed over the artery just below the cuff, or in an automated way with a microphone under the cuff.

In the oscillometric method for blood pressure measurement, the systolic and diastolic blood pressure values are based on small volume oscillations or pressure oscillations that are induced in the cuff by each heart beat. The amplitude of these volume or pressure oscillations depends on the difference between the cuff pressure and the actual arterial blood pressure. Systolic blood pressure and diastolic blood pressure are then determined as the cuff pressure where the volume or pressure oscillations have amplitudes of a certain fraction of the maximum oscillation amplitude. These fractions are typically heuristically determined.

In both the auscultatory and oscillometric method, the mean arterial pressure is typically calculated as: MAP = 2/3*DBP + 1/3*SBP.

The oscillometric and auscultatory measurement methods can be performed either during inflation of the cuff or during deflation of the cuff. Conventionally, measurements during deflation are used, in which the cuff is rapidly inflated to a level above the SBP where the blood flow in the artery under the cuff is blocked, after which cuff pressure is decreased gradually or in a stepwise manner. During deflation, the volume or pressure oscillations or the Korotkoff sounds are measured. While deflation stage measurement is well-established, an issue exists in the discomfort it introduces to the subject. In particular, the subject is exposed to a relatively high cuff pressure for a certain amount of time and pressures above a certain level can be uncomfortable and even painful, either due to the pressure exerted by the cuff itself or due to a build-up of venous blood in the clamped extremity (namely, venous pooling). The longer these pressures are applied to the subject, the higher the discomfort level is for the subject.

Another issue with utilizing the deflation based blood pressure measurement is that the process of inflating the cuff and then deflating the cuff can be considerably long, where each measurement during deflation typically takes 40 seconds to complete. Also, since a defined maximum pressure level needs to be achieved before the deflation procedure can be initiated, the subject is exposed to a maximum cuff pressure that is higher than that required for the blood pressure measurement itself. Furthermore, the inherent variability of blood pressure over time can distort a single blood pressure measurement.

Due to these issues, devices have been developed that determine oscillations during inflation of the blood pressure cuff. These devices can reduce the discomfort as blood pressure measurement may be accomplished in less time using the inflation stage, rather than the deflation stage. In order to have the measurement time as short as possible without sacrificing accuracy, the cuff inflation speed can be made pulse rate dependent such that the optimal number of oscillations (i.e. enough to assure a certain accuracy but no more) is present for determining blood pressure. Thus, once the pressure range of interest is achieved at the inflation stage, pressure relief may be initiated so as to reduce the overall measurement time and the pressure-based discomfort. An example of a device that adapts an inflation speed of a wearable cuff is described in US 9,017,264.

Nevertheless, there are also issues associated with the existing devices that adapt the inflation speed to the pulse rate of the subject. In order to achieve this, the pulse rate of the subject must be determined, which either requires an external sensor or analysis of the pressure oscillations in the cuff. In the latter case, a certain initial inflation speed can be used during which the pulse rate of the subject is determined and this initial inflation speed is changed when the pulse rate is known. The blood pressure can then be estimated from all of the pressure oscillations in the signal together (including the pressure oscillations during the pulse rate determination).

However, the change of inflation speed can induce an artifact (or transient effect) in the signal, which results in a part of the signal around the change of inflation speed being unusable for the determination of the blood pressure. This can have a negative effect on the accuracy of the blood pressure measurements, especially when the change in speed (and thus the artifact) occurs around the maximum oscillation amplitude or at oscillation amplitudes related to systolic or diastolic blood pressure. In the worst case, it may not be possible to perform a successful measurement due to the induced artifact (e.g. a measurement may stop early due to the induced oscillation) or a measurement may be incorrect due to the induced artifact, which can result in erroneous diagnoses.

## SUMMARY OF THE INVENTION

As noted above, a limitation with existing techniques for controlling a wearable cuff for blood pressure measurement is that the artifact induced by changing the speed of inflation of the wearable cuff can have a negative effect on the accuracy of blood pressure measurements acquired during the inflation of the wearable cuff. It would thus be valuable to address these limitations.

Therefore, according to a first aspect, there is provided an apparatus for controlling a wearable cuff for use in measuring blood pressure. The wearable cuff is inflatable to pressurize a measurement site of a subject. The apparatus is configured to initiate the inflation of the wearable cuff at a first inflation speed and change the first inflation speed during inflation of the wearable cuff. The first inflation speed is changed at a rate that is set to a maximum value by a limiter. The maximum value is dependent on a pulse rate acquired from the subject during inflation of the wearable cuff at the first inflation speed; and/or an amplitude of a signal indicative of pressure oscillations detected in the wearable cuff during inflation of the wearable cuff at the first inflation speed.

In some embodiments, the apparatus may be configured to change the first inflation speed to a second inflation speed during inflation of the wearable cuff. In some embodiments, the apparatus may be configured to change the first inflation speed by increasing the first inflation speed during inflation of the wearable cuff. In some embodiments, the apparatus may be configured to change the first inflation speed by decreasing the first inflation speed during inflation of the wearable cuff.

In some embodiments, the first inflation speed may be changed at a rate that is the maximum value or a rate that is less than the maximum value. In some embodiments, the first inflation speed may be changed at a rate that is variable or a rate that is constant. In some embodiments, the maximum value may be equal to or less than $16 \text{ mmHg/s}^2$.

In some embodiments, the apparatus may be configured to change the first inflation speed based on a pulse rate acquired from the subject during inflation of the wearable cuff at the first inflation speed.

In some embodiments, the maximum value may be a fixed value. In some embodiments, the maximum value may be a value that is dependent on at least one parameter. In some embodiments, the at least one parameter may comprise any one or more of a pulse rate acquired from the subject during inflation of the wearable cuff at the first inflation speed, an amplitude of a signal indicative of pressure oscillations detected in the wearable cuff during inflation of the wearable cuff at the first inflation speed, a difference between the first inflation speed and a second inflation speed, and one or more characteristics of a system comprising the wearable cuff. In some embodiments, the one or more characteristics of the system may comprise any one or more of a size of the wearable cuff and a resistance detected in the system.

In some embodiments, the apparatus may be further configured to acquire a signal indicative of pressure oscillations detected in the wearable cuff during inflation of the wearable cuff and determine a blood pressure value for the subject based on the acquired signal. In some embodiments, the acquired signal may be in a frequency range from 0.5 Hz to 5 Hz.

According to a second aspect, there is provided a system for use in measuring blood pressure. The system comprises the apparatus as described above and the wearable cuff that is inflatable to pressurize the measurement site of the subject.

According to a third aspect, there is provided a method of controlling a wearable cuff for use in measuring blood pressure. The wearable cuff is inflatable to pressurize a measurement site of a subject. The method comprises initiating the inflation of the wearable cuff at a first inflation speed and changing the first inflation speed during inflation of the wearable cuff. The first inflation speed is changed at a rate that is set to a maximum value by a limiter.

According to a fourth aspect, there is provided a computer program product comprising a computer readable medium, the computer readable medium having computer readable code embodied therein, the computer readable code being configured such that, on execution by a suitable computer or processor, the computer or processor is caused to perform the method described above.

According to the aspects and embodiments described above, the limitations of existing techniques are addressed. In particular, the above-described aspects and embodiments limit the rate at which the inflation speed is changed in order to change the inflation speed slowly. This provides control over a wearable cuff for use in measuring blood pressure in such a manner that the artifact (or transient effect) otherwise induced by a change in speed of inflation of the wearable cuff is minimized in, or even eliminated from, any signal indicative of pressure oscillations that may be detected in the wearable cuff during inflation.

Thus, the above-described aspects and embodiments provide appropriate control over the wearable cuff by limiting the rate at which the inflation speed is changed, such that the wearable cuff can be used in acquiring more accurate blood pressure measurements during inflation of the wearable cuff.

The limitations associated with the existing techniques discussed earlier are therefore addressed by way of the above-described aspects and embodiments.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 4 is a graphical illustration of example signals showing an artifact according to an existing technique;
Fig. 5 is a graphical illustration of example signals showing a reduced artifact according to an embodiment;
Fig. 6 is a graphical illustration of example signals showing a reduced artifact according to another embodiment;
Fig. 7 is a graphical illustration of example signals showing a reduced artifact according to another embodiment;
Fig. 8 is a graphical illustration of example signals showing a reduced artifact according to another embodiment; and
Fig. 9 is a graphical illustration of example signals showing a reduced artifact according to another embodiment.

DETAILED DESCRIPTION OF EMBODIMENTS

There is provided herein an apparatus for controlling a wearable cuff (or clamp unit) for use in measuring blood pressure, which overcomes the limitations with existing techniques. The wearable cuff referred to herein is inflatable to pressurize a measurement site of a subject (e.g. a patient). In this way, the wearable cuff can pressurize an artery in the measurement site of the subject. Typically, the wearable cuff can be supplied with a fluid (e.g. a gas, such as air, or any other fluid) suitable for inflating the wearable cuff. The wearable cuff can be inflatable to pressurize the measurement site of a subject (and thus an artery in the measurement site of the subject) at the pressure of the fluid in the wearable cuff.

The wearable cuff is configured to be worn on or around (e.g. wrapped around, attached to, or fastened to) the measurement site of the subject. The measurement site of the subject can be any site on the body of the subject that is suitable for use in measuring a blood pressure of the subject, such as any site on the body of the subject that comprises an artery. For example, the measurement site of the subject may be located on a limb of the subject, such as an arm (e.g. an upper arm or a forearm) of the subject. Thus, the wearable cuff can be configured to be worn on or around (e.g. wrapped around, attached to, or fastened to) a limb of the subject.

Briefly, the apparatus described herein is configured to initiate the inflation of the wearable cuff at a first inflation speed and change (or adapt) the first inflation speed during inflation (or at an inflation stage) of the wearable cuff. As described herein, the first inflation speed is changed at a rate that is limited to a maximum value. The maximum value referred to herein may also be referred to as the maximum value for the rate at which the first inflation speed is changed, the maximum value for the rate of change in the first inflation speed, or the rate limit ("RL"). Thus, the apparatus described herein is configured to limit the rate at which the first inflation speed is changed. More specifically, the apparatus can be configured to limit the rate at which the first inflation speed is changed gradually to the maximum value. It will be appreciated that a gradual change can be a change that is not immediate, instantaneous, or sudden. The apparatus can be implemented in numerous ways, with software and/or hardware, to perform the various functions described herein. In particular implementations, the apparatus can comprise a plurality of software and/or hardware modules, each configured to perform, or that are for performing, individual or multiple steps of the method described herein.

The apparatus may comprise one or more processors (such as one or more microprocessors, one or more multi-core processors and/or one or more digital signal processors (DSPs)), one or more processing units, and/or one or more controllers (such as one or more microcontrollers) that may be configured or programmed (e.g. using software or computer program code) to perform the various functions described herein. The apparatus may be implemented as a combination of dedicated hardware (e.g. amplifiers, pre-amplifiers, analog-to-digital convertors (ADCs) and/or digital-to-analog convertors (DACs)) to perform some functions and a processor (e.g. one or more programmed microprocessors, DSPs and associated circuitry) to perform other functions.

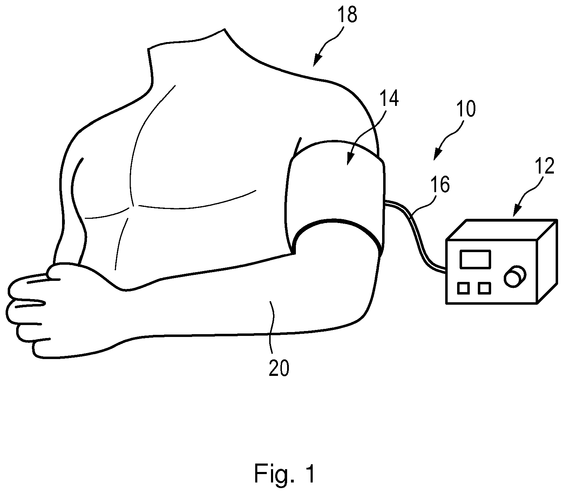

Fig. 1 illustrates the apparatus 12 in use with a wearable cuff 14 for use in measuring blood pressure, according to an example embodiment. There is thus provided a system 10 comprising the apparatus 12 and the wearable cuff 14. As mentioned earlier, the wearable cuff 14 is inflatable to pressurize the measurement site of the subject 18. In the example embodiment illustrated in Fig. 1, the measurement site of the subject 18 is located on the upper arm of the subject 18. Thus, the wearable cuff 14 is worn on or around (e.g. wrapped around, attached to, or fastened to) the upper arm of the subject 18 in this illustrated example embodiment.

As illustrated in Fig. 1, in some embodiments, the cuff 14 may be coupled with or connected to the apparatus 12 via at least one supply line (or at least one supply tube) 16, which may also be referred to as at least one pressure supply line (or at least one pressure supply tube) 16. The at least one supply line 16 can be arranged for pressurizing the wearable cuff 14 and, consequently, the measurement site of the subject 18. The at least one supply line 16 may be provided for inflating and/or deflating the wearable cuff 14. As an alternative to the at least one supply line 16, in other embodiments (not illustrated), the apparatus 12 can be coupled directly to (e.g. mounted directly on) the wearable cuff 14. As mentioned earlier, the wearable cuff 14 can be supplied with any fluid suitable for inflating the wearable cuff 14.

Although not illustrated in Fig. 1, in some embodiments, the system 10 may comprise a pump. The pump can be controllable to inflate the wearable cuff 14 in the manner described herein. In some embodiments, the apparatus 12 described herein may comprise the pump. Alternatively or in addition, a pump may be external to (e.g. separate to or remote from) the apparatus 12. A pump can thus be any pump that is controllable to inflate the wearable cuff 14. In some embodiments, the pump can be controllable by a controller of the apparatus 12 to inflate the wearable cuff 14 in the manner described herein. The controller of the apparatus 12 may communicate with and/or connect to the pump in any suitable way to control the pump.

Although also not illustrated in Fig. 1, in some embodiments, the system 10 may comprise a deflation valve. The deflation valve can be controllable to deflate the wearable cuff 14. In some embodiments, the apparatus 12 described herein may comprise the deflation valve. Alternatively or in addition, a deflation valve may be external to (e.g. separate to or remote from) the apparatus 12. A deflation valve can thus be any valve that is controllable to deflate the wearable cuff 14. In some embodiments, the deflation valve can be controllable by the controller of the apparatus 12 to deflate the wearable cuff 14. The controller of the apparatus 12 may communicate with and/or connect to the deflation valve in any suitable way to control the deflation valve.

Although also not illustrated in Fig. 1, in some embodiments, the system 10 may comprise at least one pressure sensor. The at least one pressure sensor can be configured to measure the pressure in the wearable cuff 14. In some embodiments, the apparatus 12 described herein may comprise the at least one pressure sensor configured to measure the pressure in the wearable cuff 14. Alternatively or in addition, at least one pressure sensor external to (e.g. separate to or remote from) the apparatus 12 may be configured to measure the pressure in the wearable cuff 14. For example, in some embodiments, the wearable cuff 14 itself may comprise at least one pressure sensor configured to measure the pressure in the wearable cuff 14. In some embodiments, the at least one pressure sensor can be controllable by the controller of the apparatus 12 to measure the pressure in the wearable cuff 14. The controller of the apparatus 12 may communicate with and/or connect to the at least one pressure sensor in any suitable way to control the at least one pressure sensor.

Although also not illustrated in Fig. 1, in some embodiments, the system 10 may comprise a communications interface (or communications circuitry). In some embodiments, the apparatus 12 described herein may comprise a communications interface. Alternatively or in addition, the communications interface may be external to (e.g. separate to or remote from) the apparatus 12. The communications interface can be for enabling the apparatus 12, or components of the apparatus 12, to communicate with and/or connect to one or more other components, sensors, interfaces, devices, or memories (such as any of those described herein). For example, the communications interface can be for enabling the controller of the apparatus 12 to communicate with and/or connect to any one or more of the pump, the deflation valve, and the at least one pressure sensor described earlier. The communications interface may enable the apparatus 12, or components of the apparatus 12, to communicate and/or connect in any suitable way. For example, the communications interface may enable t the apparatus 12, or components of the apparatus 12, to communicate and/or connect wirelessly, via a wired connection, or via any other communication (or data transfer) mechanism. In some wireless embodiments, for example, the communications interface may enable the apparatus 12, or components of the apparatus 12, to use radio frequency (RF), Bluetooth, or any other wireless communication technology to communicate and/or connect.

Although also not illustrated in Fig. 1, in some embodiments, the system 10 may comprise a memory. In some embodiments, the apparatus 12 described herein may comprise the memory. Alternatively or in addition, the memory may be external to (e.g. separate to or remote from) the apparatus 12. The controller of the apparatus 12 may be configured to communicate with and/or connect to the memory. The memory may comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM), and electrically erasable PROM (EEPROM). In some embodiments, the memory can be configured to store program code that can be executed by a processor to cause the apparatus 12 to operate in the manner described herein.

Alternatively or in addition, in some embodiments, the memory can be configured to store information required by or resulting from the method described herein. For example, in some embodiments, the memory may be configured to store any one or more of the inflation speeds described herein, the rate at which the first inflation speed is changed, the maximum value for this rate, one or more blood pressure measurements acquired during use of the apparatus 12 in the manner described herein, or any other information, or any combination of information, required by or resulting from the method described herein. In some embodiments, the controller of the apparatus 12 can be configured to control the memory to store information required by or resulting from the method described herein.

Although also not illustrated in Fig. 1, the system 10 may comprise a user interface. In some embodiments, the apparatus 12 described herein may comprise the user interface. Alternatively or in addition, the user interface may be external to (e.g. separate to or remote from) the apparatus 12. The controller of the apparatus 12 may be configured to communicate with and/or connect to a user interface. The user interface can be configured to render (or output, display, or provide) information required by or resulting from the method described herein. For example, in some embodiments, the user interface may be configured to render (or output, display, or provide) one or more of the inflation speeds described herein, the rate at which the first inflation speed is changed, the maximum value for this rate, one or more blood pressure measurements acquired during use of the apparatus 12 in the manner described herein, or any other information, or any combination of information, required by or resulting from the method described herein. Alternatively or in addition, the user interface can be configured to receive a user input. For example, the user interface may allow a user to manually enter information or instructions, interact with and/or control the apparatus 12. Thus, the user interface may be any user interface that enables the rendering (or outputting, displaying, or providing) of information and, alternatively or in addition, enables a user to provide a user input. In some embodiments, the controller of the apparatus 12 can be configured to control the user interface to operate in the manner described herein.

For example, the user interface may comprise one or more switches, one or more buttons, a keypad, a keyboard, a mouse, a touch screen or an application (for example, on a smart device such as a tablet, a smartphone, or any other smart device), a display or display screen, a graphical user interface (GUI) such as a touch screen, or any other visual component, one or more speakers, one or more microphones or any other audio component, one or more lights (such as light emitting diode LED lights), a component for providing tactile or haptic feedback (such as a vibration function, or any other tactile feedback component), an augmented reality device (such as augmented reality glasses, or any other augmented reality device), a smart device (such as a smart mirror, a tablet, a smart phone, a smart watch, or any other smart device), or any other user interface, or combination of user interfaces. In some embodiments, the user interface that is controlled to render information may be the same user interface as that which enables the user to provide a user input.

Although also not illustrated in Fig 1, the apparatus 12 may comprise a battery or other power supply for powering the apparatus 12 or means for connecting the apparatus 12 to a mains power supply. It will also be understood that the apparatus 12 may comprise any other component to those described herein or any combination of components.

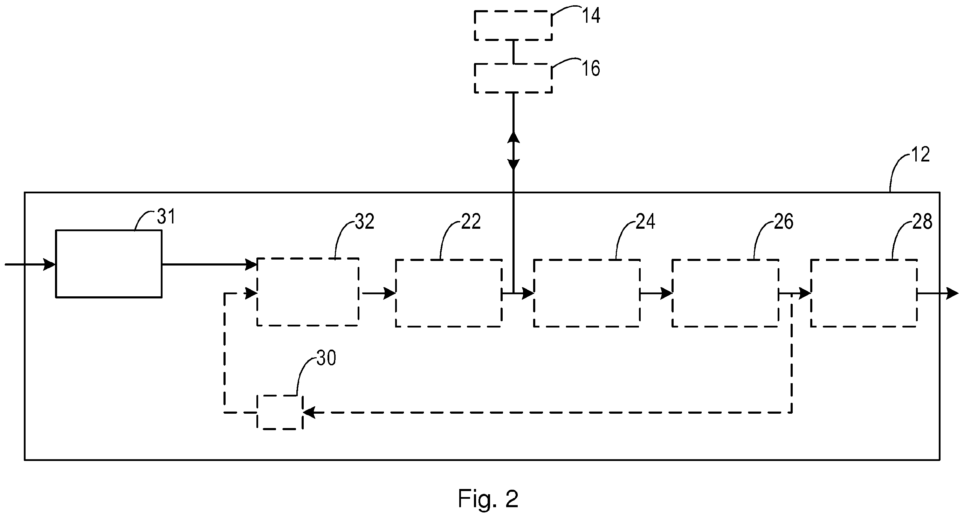

Fig. 2 illustrates in more detail the apparatus 12 (as described earlier with reference to Fig. 1) for controlling a wearable cuff 14 according to an example embodiment. With reference to Fig. 2, according to this example embodiment, the apparatus 12 may comprise any one or more of a pump 22 (such as the pump described earlier with reference to Fig. 1, which can be connected to the subject 18 via the wearable cuff 14 and optionally also the at least one supply tube 16), at least one pressure sensor 24 (such as the at least one pressure sensor described earlier with reference to Fig. 1), an analog filter 26, an oscillation filter 28, a converter 30, a limiter 31, and a controller or pump controller 32 (such as that mentioned earlier with reference to Fig. 1).

With reference to Fig. 2, in some embodiments, the at least one pressure sensor 24 can be configured to measure the pressure in the wearable cuff 14. The converter 30 may receive the pressure measured in the wearable cuff 14 from the at least one pressure sensor 24. In some embodiments, the converter 30 may be configured to acquire a measured (or actual) inflation rate of the wearable cuff 14 by converting the pressure measured in the wearable cuff 14 into a pressure rate. For example, the converter 30 may be configured to take a derivative (or, more specifically, a time derivative d/dt) of the pressure measured in the wearable cuff 14 to convert the measured pressure into a pressure rate. The pressure rate can be indicative of the measured (or actual) inflation rate.

As described earlier, the inflation of the wearable cuff 14 is initiated at a first inflation speed and this first inflation speed is changed during inflation of the wearable cuff 14. In the illustrated example embodiment of Fig. 2, the limiter 31 is configured to limit the rate at which the first inflation speed is changed to a maximum value. In other words, the limiter 31 is configured to limit the rate of change in the first inflation speed to the maximum value. More specifically, the limiter 31 can be configured to limit the rate at which the first inflation speed is changed (or the rate of change in the first inflation speed) gradually to the maximum value. As mentioned earlier, it will be appreciated that a gradual change can be a change that is not immediate, instantaneous, or sudden. The maximum value for the rate at which the first inflation speed is changed can be set by the limiter 31. The limiter 31 may thus also be referred to as an inflation speed change limiter (ISCL) or a rate limiter (RL). In effect, the limiter 31 can be configured to translate a target rate of change (e.g. which has a steep change) in the first inflation speed, which the limiter 31 may receive as an input, to a target rate of change in the first inflation speed that is limited to the maximum value (such that the change in the first inflation speed occurs slowly). The target rate of change in the first inflation speed that is limited to the maximum value can be referred to as the limited target rate of change in the first inflation speed.

The controller 32 can be configured to receive the limited target rate of change in the first inflation speed from the limiter 31. The controller 32 is configured to initiate the inflation of the wearable cuff 14 at a first inflation speed and change the first inflation speed during inflation of the wearable cuff 14 in the usual way but at a rate that is limited to the maximum value, i.e. according to the limited target rate of change in the first inflation speed that the controller 32 receives from the limiter 31. For example, in some embodiments, the controller 32 can also be configured to receive the measured (or actual) inflation rate of the wearable cuff 14 from the converter 30. According to these embodiments, the controller 32 can be configured to translate the limited target rate of change in the first inflation speed received from the limiter 31 and the measured (or actual) inflation rate received from the convertor 30 into a control signal for output to the pump 22.

In some embodiments, for example, the controller 32 can be configured to compare the limited target rate of change in the first inflation speed and the measured (or actual) inflation rate. In this way, the controller 32 can be configured to determine a difference between the measured (or actual) inflation rate and the limited target rate of change in the first inflation speed that the controller 32 receives from the limiter 31. In these embodiments, the controller 32 can be configured to control the pump 22 based on the difference between the measured (or actual) inflation rate and the limited target rate of change in the first inflation speed. In some of these embodiments, the controller 32 can be configured to output a control signal to the pump 22 that makes the difference between the limited target rate of change in the first inflation speed and the measured inflation rate as small as possible. In this way, a sudden change (e.g. any sudden step) in the first inflation speed and thus a corresponding artifact is minimized or even eliminated. In a steady state, the difference can be zero. In some embodiments, the change in the first inflation speed can be a change in the first inflation speed from a speed used during pulse rate determination to a pulse rate dependent speed.

The control signal from the controller 32 is thus received by the pump 22 and is used to control the pump to inflate the wearable cuff 14 in the manner described herein, which reduces or even eliminates the artifact that is otherwise induced in a pressure signal indicative of the pressure in the wearable cuff 14. The pump 22 may not follow the control signal exactly but instead can have a certain system response, $H_{pump}(s)$. The system response of the pump can, for example, have some delay and/or overshoot (or ringing) on a step input. Generally, the dynamic pump response may be non-linear over pressure and may also depend on its operating condition (e.g. driving signal (such as a driving voltage), rotation speed (e.g. in the case of a centrifugal pump)), the actual pump response may thus also be a function of (or depend on) the pressure, $H_{pump}(s,p)$.

The pneumatics in the system 10 (which consist of the wearable cuff 14 that is worn on or around the subject 18 and optionally also the at least one supply tube 16) has its own system response based on the input received from the pump 22. In the simplest form, the pneumatics response can be modeled as a simple resistance/compliance combination, $H_{pneumatic}(s)$. Generally, this pneumatics response is non-linear over pressure and thus the actual pneumatics response may be a function of (or depend on) pressure, $H_{pneumatic}(s,p)$.

As mentioned earlier, the at least one pressure sensor 24 can be configured to measure the pressure in the wearable cuff 14. The at least one pressure sensor 24 may also be configured to convert the measured pressure into the electrical domain. For example, the at least one pressure sensor 24 may be configured to convert the measured pressure into an electrical signal. The electrical signal may then pass an analog filter 26, such as an analog electrical low pass filter (e.g. an anti-aliasing filter). The analog filter 26 has a certain filter response $H_{analogfilter}(s)$. The analog filter 26 may operate as a first-order resistor-capacitor (RC) filter. The electrical signal may the pass an oscillation filter 28. The oscillation filter (such as a digital oscillation filter) 28 can be configured to remove the inflation ramp from the pressure oscillations. The oscillation filter 28 has a certain filter response $H_{oscfilter}(s)$ on an inflation rate change. This filter response can be known exactly where the filter is implemented as a digital filter.

The total system response from a change in the first inflation speed to a result in the oscillation signal is a combination of all of the above-described responses. In the frequency domain, this can be written as:

$$H_{Total}(s,p) = H_{controller}(s) \cdot H_{pump}(s,p) \cdot H_{pneumatic}(s,p) \cdot H_{analogfilter}(s) \cdot H_{oscfilter}(s).$$

Measures can be taken to make the system response independent of pressure. For example, the measures may comprise modeling the pressure dependent components and compensating for pressure in a digital way. In this way, $H_{Total}(s,p)$ reduces to $H_{Total}(s)$, i.e. the dependence on pressure is removed.

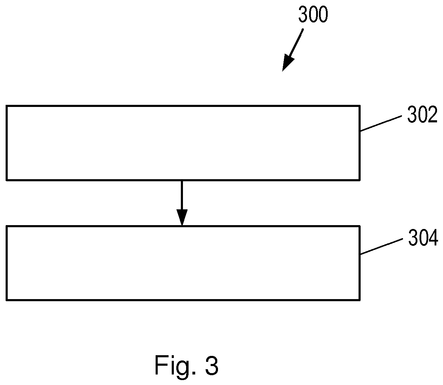

Fig. 3 illustrates a method 300 of controlling a wearable cuff 14 for use in measuring blood pressure according to an embodiment. As mentioned earlier, the wearable cuff 14 is inflatable to pressurize a measurement site 20 of a subject 18. The method 300 can generally be performed by or under the control of the controller 32 of the apparatus 12 described earlier. At block 302, the inflation of the wearable cuff 14 is initiated at a first inflation speed. At block 304, the first inflation speed is changed during inflation of the wearable cuff 14. For example, in some embodiments, the apparatus 12 may be configured to change the first inflation speed to a second inflation speed during inflation of the wearable cuff 14. As mentioned earlier, the first inflation speed is changed at a rate that is limited to a maximum value, which can be set by the limiter 31.

In some embodiments, the apparatus 12 may be configured to change the first inflation speed by increasing the first inflation speed during inflation of the wearable cuff 14. In other embodiments, the apparatus 12 may be configured to change the first inflation speed by decreasing the first inflation speed during inflation of the wearable cuff. The apparatus 12 may be configured to determine whether to change the first inflation speed by increasing the first inflation speed during inflation of the wearable cuff or by decreasing the first inflation speed during inflation of the wearable cuff depending on the value of the first inflation speed.

In a typical implementation, the change in inflation speed is instantaneous, resulting in a step or "a step change" in the inflation speed. This sudden change in inflation speed has been found to be the cause of an artifact induced in the pressure signal indicative of the pressure in the wearable cuff 14. This artifact propagates to the signal containing the pressure oscillations and thus introduces errors in blood pressure measurements acquired by analyzing the pressure oscillations measured in the wearable cuff 14. The manner in which the apparatus 12 controls inflation of the wearable cuff 14 by changing the first inflation speed during inflation of the wearable cuff 14 at a rate that is limited to a maximum value, minimizes or even eliminates the artifact in the pressure signal indicative of the pressure in the wearable cuff 14 and thus from pressure oscillations measured in the wearable cuff 14 to thereby allow more accurate blood pressure measurements to be acquired.

In more detail, in order to have a valid pressure signal (or pressure envelope), a change in pressure in the wearable cuff 14 due to influences other than pressure oscillations (such as the artifact due to a change in the first inflation speed) need to be small or negligible compared to the pressure change within a valid pressure oscillation in the wearable cuff 14. A pressure oscillation in the wearable cuff 14 has a certain minimum value ($A_{min}$) and maximum value ($A_{max}$). The amplitude of an oscillation is calculated as the maximum value ($A_{max}$) minus the minimum value ($A_{min}$). If both the maximum value ($A_{max}$) and the minimum value ($A_{min}$) are shifted with the same amount, the resulting amplitude will be unaffected. If only one of the maximum value ($A_{max}$) and the minimum value ($A_{min}$) is changed, the oscillation amplitude will be distorted.

For example, consider a pressure oscillation in the wearable cuff 14 with a maximum at a first time, t = 0 seconds, and a minimum at a later second time, t = 0.5 seconds. A change in a target inflation speed starts at t>0, resulting in a pressure artifact (or transient) over time. The maximum value of the pressure oscillation (at t = 0 seconds) will be unaffected. However, the minimum value (at t = 0.5 seconds) of the pressure oscillation will be affected. The amount of the effect depends on the degree by which the pressure changes in the 0.5 seconds between the minimum value and maximum value of the pressure oscillation.

The pressure induced (or the artifact) in the pressure signal due to a change in the target inflation speed of the wearable cuff 14 is a function of the system response $H_{Total}(s)$ and the change in the target inflation speed itself. In the worst case, the change in the target inflation speed ("ΔInflationSpeed") is a step in the target inflation speed ("$X_{step}$"), which is equal to the target inflation speed after the change in inflation speed (which is referred to herein as the second inflation speed) minus the target inflation speed before the change in inflation speed (which is referred to herein as the first inflation speed). The induced pressure due to a change in the first inflation speed of the wearable cuff 14 can be written as the convolution of the system response $H_{Total}$ and the step in inflation speed $X_{step}$, as follows:

$$\text{Induced pressure} = (H_{total} * X_{step})\,(t). \tag{1}$$

The maximum in this induced pressure is therefore a certain scaling factor $M_1$ (which is related to the system response $H_{Total}$) times the change in the first inflation speed, as follows:

$$\text{Maximum induced pressure} = M_1 \cdot \Delta\text{InflationSpeed}. \tag{2}$$

The induced pressure waveform due to a change in the first inflation speed of the wearable cuff 14 can (dependent on the system) have a relatively long duration (e.g. in the order of seconds) and can span multiple pressure oscillations in the pressure signal, where a single pressure oscillation can be present during only a part of the induced pressure waveform. In the short time frame of a single pressure oscillation, the induced pressure can have a maximum change of max($\text{dpressure}_{induced}$/dt) multiplied by the duration of the short time frame.

In order to have no effect on the pressure oscillation envelope, the maximum induced pressure needs to be much smaller than, or negligible compared to, the change in the oscillation signal:
Maximum induced pressure < change in oscillation signal,

$$\text{max}(\text{dpressure}_{\text{induced}}/\text{dt}) \cdot \text{duration}_{\text{maxtominoscillation}} < \text{change in oscillation} \qquad (3)$$

$$\text{amplitude},$$

where $\text{max}(\text{dpressure}_{\text{induced}}/\text{dt})$ is again a system dependent factor (which will be denoted as $M_2$) times the change in the first inflation speed:

$$\text{max}(\text{dpressure}_{\text{induced}}/\text{dt}) = M_2 \cdot \Delta\text{InflationSpeed}. \qquad (4)$$

Between the minimum value ($A_{\text{min}}$) and maximum value ($A_{\text{max}}$) of an actual pressure oscillation in the wearable cuff 14, the pressure changes from the minimum value ($A_{\text{min}}$) to the maximum value ($A_{\text{max}}$):

$$\text{change in oscillation pressure} = A_{\text{max}} - A_{\text{min}}. \qquad (5)$$

When Equation (4) and Equation (5) are substituted into Equation (3), it can be seen that:

$$M_2 \cdot \Delta\text{InflationSpeed} \cdot \text{duration}_{\text{maxtominoscillation}} < A_{\text{max}} - A_{\text{min}}. \qquad (6)$$

The duration between the minimum value ($A_{\text{min}}$) and maximum value ($A_{\text{max}}$) of a pressure oscillation in the wearable cuff 14 is a fraction (c) of the pulse rate (PR), as follows:

$$\text{duration}_{\text{maxtominoscillation}} = c/(\text{PR}/60). \qquad (7)$$

The fraction c in Equation (7) is not 50% due to an asymmetry of the pressure pulse between rising and falling. When substituting Equation (7) into Equation (6):

$$M_2 \cdot \Delta\text{InflationSpeed} \cdot c/(\text{PR}/60) < A_{\text{max}} - A_{\text{min}}, \qquad (8)$$

$$M_2 \cdot \Delta\text{InflationSpeed} < (A_{\text{max}} - A_{\text{min}}) \cdot (\text{PR}/60)/c, \qquad (9)$$

$$M_2 \cdot \Delta\text{InflationSpeed} <= k \cdot (A_{\text{max}} - A_{\text{min}}) \cdot (\text{PR}/60)/c, \qquad (10)$$

$$M_2 \cdot \Delta\text{InflationSpeed} <= K_1 \cdot (A_{\text{max}} - A_{\text{min}}) \cdot \text{PR}. \qquad (11)$$

The requirement for the change in induced pressure in the oscillation signal to be small is shown in Equation (11). With a desired change in the first inflation speed known and the pulse rate and amplitude being measurable, the only potential unknowns in Equation (11) are $M_2$ and $K_1$. $M_2$ is a system ($H_{\text{total}}$) related parameter that is fixed and can be determined a priori (e.g. during system design). The parameter $K_1$ depends on c (which is the worst case pressure oscillation) and k (<1), which describes the extent to which the induced pressure in the oscillation signal must be smaller than a valid pressure oscillation in the wearable cuff 14. This parameter can be determined experimentally. Therefore, at the moment of change of the first inflation speed, all parameters are known and it can be predicted whether a desired change in the first inflation speed results in an induced pressure in the oscillation signal that will affect the oscillation amplitude.

As shown in Equation (11), the extent to which the induced pressure in the oscillation signal must be smaller than a valid pressure oscillation in the wearable cuff 14 depends on the amplitude of a pressure oscillation in the wearable cuff 14 and the pulse rate of the subject 18. This is logical since smaller pressure oscillation amplitudes are more disturbed by an induced pressure than larger pressure oscillation amplitudes. The same holds for the dependence on pulse rate. The greater the number of pressure oscillations in the period of the induced pressure, the lesser the effect on the amplitude of each of those pressure oscillations. Similarly, the lower the number of pressure oscillations in the period of the induced pressure, the larger the effect on the amplitude of each of those pressure oscillations.

It can be seen from Equation (11) that the induced oscillation is considered small if $(M_2 \cdot \Delta InflationSpeed)$ is smaller than $K_1 \cdot (A_{max}-A_{min}) \cdot PR$. By ensuring that the first inflation speed changes slowly, rather than there being a sudden step in inflation speed, the left side of Equation (11) can be changed such that the induced pressure oscillation can be considered small. This is achieved, as described earlier, by changing the first inflation speed during inflation of the wearable cuff 14 at a rate that is limited to a maximum value (at block 304 of Fig. 3). Thus, the rate at which the first inflation speed is changed during inflation of the wearable cuff 14 is limited. This saturates the derivative of the target pressure rate signal. By rate limiting the change in the first inflation speed in this way, the maximum change in the oscillation pressure max(dpressureinduced/dt) and hence $M_2 \cdot \Delta InflationSpeed$ are upper bounded by a value that is dependent on the system $H_{total}$ and:

$$M_2 \cdot \Delta InflationSpeed \; < f(RL, H_{total}) \qquad (12)$$

Here, the maximum value for the rate at which the first inflation speed is changed (or the rate limit) is denoted by "RL". $\Delta InflationSpeed$ is not part of this equation, as the maximum value for the rate at which the first inflation speed is changed (RL) limits the speed at which the step is made. $f(RL, H_{total})$ can be further simplified to:

$$f(RL, H_{total}) = M_3 \cdot RL \qquad (13)$$

where $M_3$ is a system dependent factor. By substituting Equation (13) in Equation (11):

$$M_3 \cdot RL <= K_1 \cdot (A_{max} - A_{min}) \cdot PR, \qquad (14)$$

$$RL <= K_1/M_3 \cdot (A_{max} - A_{min}) \cdot PR. \qquad (15)$$

From equation (15), it can be seen that choosing an appropriate fixed value for the maximum rate of change in the first inflation speed (RL) results in the induced artifact begin small compared to the pressure oscillations. That is, the maximum value for the rate at which the first inflation speed is changed (RL) may be chosen based on the worst case oscillation amplitude $(A_{max} - A_{min})$ and the worst case pulse rate (PR). Alternatively, the maximum value for the rate at which the first inflation speed is changed (RL) can be made proportional to both the oscillation amplitude $(A_{max} - A_{min})$ and the oscillation frequency (i.e. the pulse rate (PR)) of a signal indicative of pressure oscillations detected in the wearable cuff 14:

$$RL = K_2 \cdot (A_{max} - A_{min}) \cdot PR. \qquad (16)$$

In Equation (16), the factor $K_2$ depends on the system transfer and may be chosen such that $K_2 <= K_1/M_3$. When choosing the factor $K_2$, the limiter 31 needs the amplitude and pulse rate as an input. These values can be determined from the signal indicative of pressure oscillations detected in the wearable cuff 14 during inflation of the wearable cuff 14 with standard signal processing techniques. In some embodiments, the factor $K_2$ can be adaptive. For example, the factor $K_2$ can be dependent on system parameters (such as on the resistance of at least one supply tube 16 and/or the compliance of the wearable cuff 14).

Alternatively or in addition to the maximum value for the rate at which the first inflation speed is changed (RL) depending on the oscillation amplitude and/or the oscillation frequency (as in Equation (16)), the maximum value for the rate at which the first inflation speed is changed (RL) can also incorporate the dependence on the magnitude of the change in the first inflation speed (or the inflation speed step size, "$\Delta InflationSpeed$").

Thus, in some embodiments, the maximum value for the rate at which the first inflation speed is changed (RL) may be adaptive. For example, in some embodiments, the maximum value for the rate at which the first inflation speed is changed (RL) may be a value that is dependent on at least one parameter. As mentioned earlier, examples of the at least one parameter include, but are not limited to, any one or more of a pulse rate acquired from the subject 18 (or a frequency of a signal indicative of pressure oscillations detected in the wearable cuff 14) during inflation of the wearable cuff 14 at the first inflation speed, an amplitude of a signal indicative of pressure oscillations (or an oscillation amplitude of beats) detected in the wearable cuff 14 during inflation of the wearable cuff 14 at the first inflation speed, a difference between the first inflation speed and a second inflation speed (or the step size in speed), and one or more characteristics of a system 10 comprising the wearable cuff 14. In some embodiments, the one or more characteristics of the system 10 may comprise any one or more of a size of the wearable cuff 14 and a resistance detected in the system 10.

In other embodiments, the maximum value for the rate at which the first inflation speed is changed (RL) may be a fixed value (or fixed rate).

In some embodiments, the first inflation speed may be changed at a rate that is constant. Alternatively, in other embodiments, the first inflation speed may be changed at a rate that is variable. In some embodiments, the apparatus 12 may be configured to change the first inflation speed based on a pulse rate acquired from the subject 18 during inflation of the wearable cuff 14 at the first inflation speed. The pulse rate may, for example, be acquired from the subject 18 by analyzing a pressure signal indicative of the pressure in the wearable cuff 14 during inflation of the wearable cuff 14 at the first inflation speed to determine the pulse rate of the subject 18. For example, the pulse rate may be determined as a frequency of the pressure signal indicative of pressure detected in the wearable cuff 14. By changing the first inflation speed at a value proportional to the oscillation amplitude and pulse rate of the subject 18, it is possible to make a duration for any blood pressure measurement as short as possible without introducing an artifact.

In some embodiments, the first inflation speed may be changed at a rate that is the maximum value for the rate at which the first inflation speed is changed (RL). Alternatively, in other embodiments, the first inflation speed may be changed at a rate that is less than the maximum value for the rate at which the first inflation speed is changed (RL). In some embodiments, the maximum value for the rate at which the first inflation speed is changed (RL) may be in the range from 1 to 16 mmHg/s$^2$.

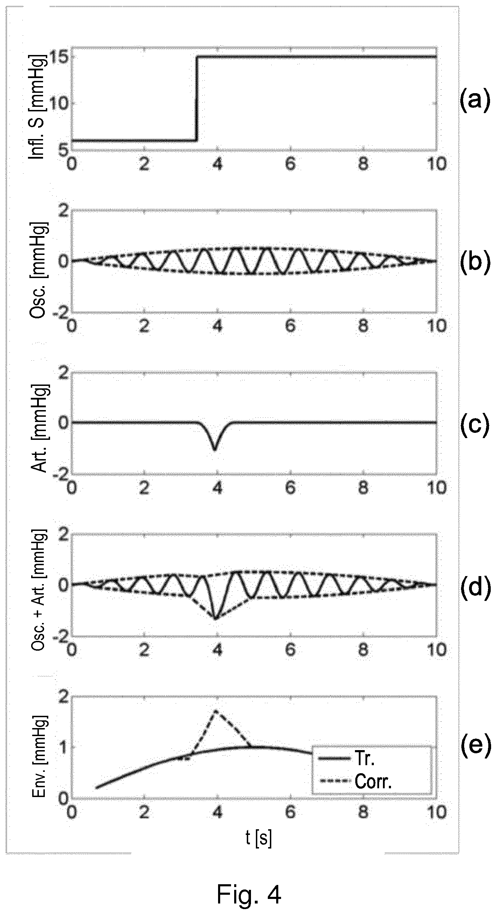

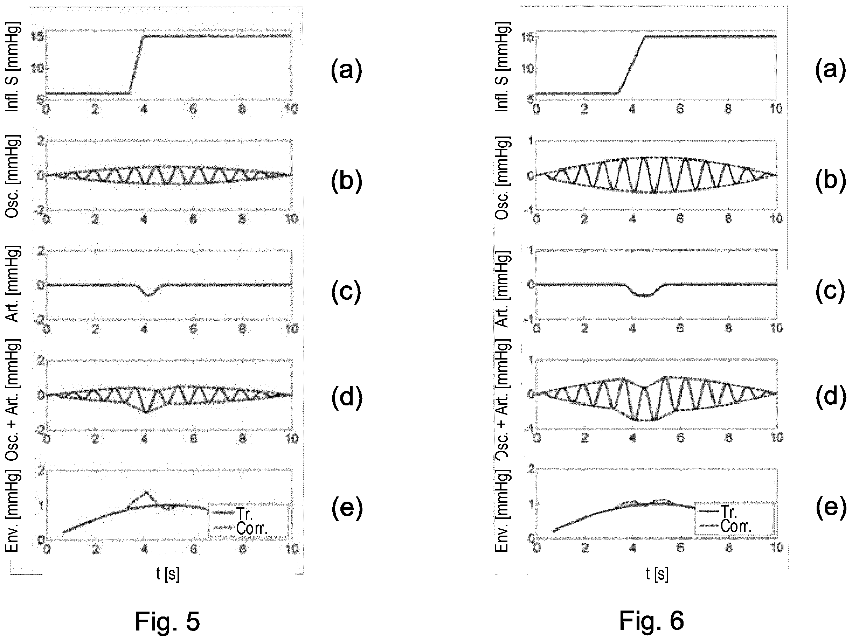

Fig. 4 is a graphical illustration of example signals showing an artifact due to a change in a speed of inflation of a wearable cuff 14 during blood pressure measurement according to an existing technique. Figs. 5-9 are graphical illustrations of example signals showing a reduced artifact according to some embodiments that implement the apparatus 12 described herein. Figs. 4-9 will now be described to illustrate the minimization of the artifact induced due to a change in a speed of inflation of a wearable cuff 14 that can be achieved using the apparatus 12 described herein.

Fig. 4(a) is a graphical illustration of the inflation speed "Infl. S" over time according to the existing technique. As illustrated in Fig. 4(a), the inflation speed changes from a first inflation speed of 6 mmHg/s to a second inflation speed of 15mmHg/s at t=3.5s. Fig. 4(b) is a graphical illustration of an undisturbed (or undistorted) pressure signal "Osc." indicative of pressure oscillations in the wearable cuff 14 over time during inflation of the wearable cuff 14. Fig. 4(c) is a graphical illustration of an artifact "Art." induced in the pressure signal over time due to the change in the speed of inflation. This artifact is the response of the system 10 comprising the wearable cuff 14 excited by the step size in speed (which is 9 mmHg/s in this case).

Fig. 4(d) is a graphical illustration of the disturbed (or corrupted) pressure signal, where the pressure signal is disturbed (or corrupted) by the artifact. Thus, Fig. 4(d) graphically illustrates the combined artifact and pressure signal, showing that the pressure oscillations in the wearable cuff 14 are disturbed by the artifact. This disturbance by the artifact results in disturbed top and bottom envelopes, illustrated by the dashed lines. Fig. 4(e) is a graphical illustration of the resulting envelope "Env." of the (original or true) undisturbed pressure signal "Tr." and the disturbed (or corrupted) pressure signal "Corr.". From the description of Figs. 5-9 that follow, it will be seen that through use of the apparatus 12 described herein, the artifact induced due to a change in a speed of inflation of a wearable cuff 14 can be minimized compared to existing techniques such as that illustrated in Fig. 4.

Figs. 5 and 6 illustrate the same as Fig. 4, except that the first inflation speed is changed at a rate that is limited to a maximum value of 16 mmHg/s$^2$ in the example embodiment illustrated in Fig. 5 and the first inflation speed is changed at a rate that is limited to a maximum value of 8 mmHg/s$^2$ in the example embodiment illustrated in Fig. 6. As can be seen from Fig. 5 compared to Fig. 4, when the rate at which the first inflation speed changes is limited to a maximum value of 16 mmHg/s$^2$, the error in the disturbed (or corrupted) pressure signal is much less than when using no limit on the rate as in Fig. 4. Similarly, as can be seen from Fig. 6 compared to Fig. 4, when the rate at which the first inflation speed changes is limited to a maximum value of 8 mmHg/s$^2$, the error in the disturbed (or corrupted) pressure signal is less than when using a limit on the rate of 16 mmHg/s$^2$ as in Fig. 5, much less than when using no limit on the rate as in Fig. 4 and is, in fact, practically eliminated.

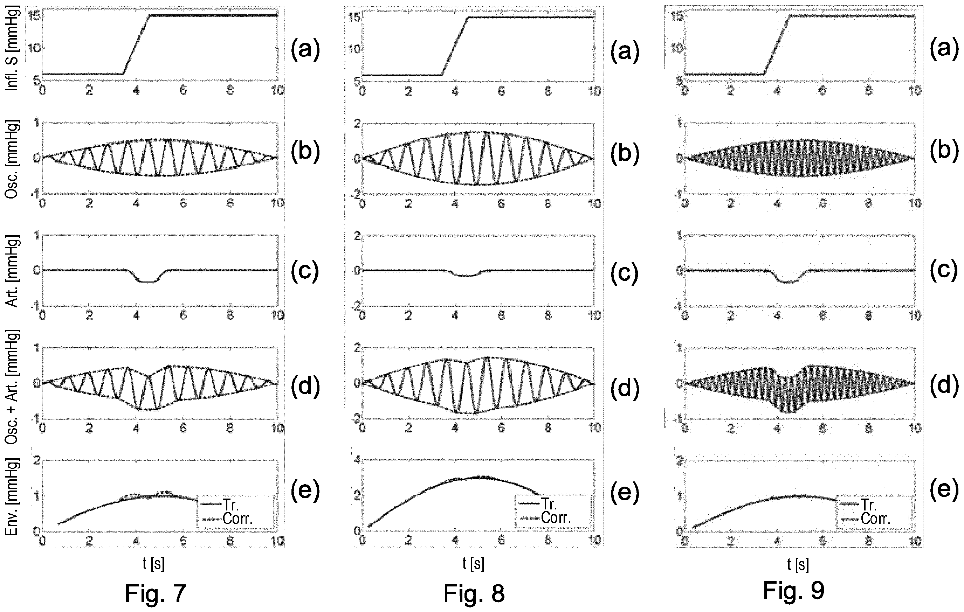

Figs. 7-9 also illustrate the same as Fig. 4, except that the first inflation speed is changed at a rate that is limited to a maximum value of 8 mmHg/s$^2$ in the example embodiments illustrated in Figs. 7-9. Figs 7-9 illustrate the effect of this rate limit on three different oscillations settings, namely on 1 mmHg peak-peak oscillation amplitude and 70 beats per minute in Fig. 7, on 3 mmHg peak-peak oscillation amplitude and 70 beats per minute in Fig. 8, and on 1 mmHg peak-peak oscillation amplitude and 210 beats per minute in Fig. 9.

As can be seen from Figs. 7-9 compared to Fig. 4, when the rate at which the first inflation speed changes is limited to a maximum value of 8 mmHg/s$^2$, the error in the disturbed (or corrupted) pressure signal is much less than when using no limit on the rate as in Fig. 4 for each of the different oscillations settings. As can also be seen from Figs.

7-9, the artifact is the same for each of the different oscillations settings. However, the effect on the resulting pressure envelope is different for each of the different oscillations settings based on the amplitude and frequency of the oscillations in the pressure signal. Where the oscillations are small and/or slow compared to the artifact, the artifact can still disturb the signal (as illustrated in Fig. 7). Where the artifact is small compared to the oscillations (as illustrated in Fig. 8) or where the oscillations are fast compared to the artifact (as illustrated in Fig 9), then the resulting pressure envelope is affected less. In case of the fast oscillations (as illustrated in Fig. 9), the oscillations show a dip due to the artifact but the resulting pressure envelope is only minimally affected by the artifact as the artifact affects both the minima and maxima of each oscillation.

Thus, it can be seen from Figs. 5-9 that the apparatus 12 described herein that limits the rate at which the first inflation speed of a wearable cuff 14 changes can reduce or even eliminate the artifact (or transient effect), which would otherwise be induced by the change in the first inflation speed, from signals indicative of pressure oscillations in the wearable cuff 14 during inflation compared to existing techniques, such as that illustrated in Fig. 4.

In any of the embodiments described herein, the apparatus 12 can be further configured to acquire a signal indicative of pressure oscillations detected in the wearable cuff 14 during inflation of the wearable cuff 14. In these embodiments, the apparatus 12 (e.g. the controller 32 of the apparatus 12) can also be further configured to determine (or measure) a blood pressure value for the subject 18 based on the acquired signal. Alternatively, a module external to (e.g. separate to or remote from) the apparatus 12 may be configured to acquire a signal indicative of pressure oscillations detected in the wearable cuff 14 during inflation of the wearable cuff 14 and determine (or measure) a blood pressure value for the subject 18 based on the acquired signal. Thus, the apparatus 12 (or an external module) can be configured to determine a blood pressure value for the subject 18 during inflation of the wearable cuff 14 according to some embodiments.

As the blood pressure value for the subject 18 is determined based on a signal acquired during inflation (or at the inflation stage) of the wearable cuff 14, the apparatus 12 is for use in inflation-based blood pressure measurements. More specifically, the apparatus 12 is for use in inflation-based non-invasive blood pressure (iNIBP) measurements. In some embodiments, the acquired signal may be in a frequency range that corresponds to a heart rate range of 30 beats/min to 300 beats/min. For example, the acquired signal may be in a frequency range from 0.5 Hz to 5 Hz according to some embodiments.

In any of the embodiments described herein, at least one or all of the steps that the apparatus 12 is configured to perform can be automated.

There is also provided a computer program product comprising a computer readable medium. The computer readable medium has computer readable code embodied therein. The computer readable code is configured such that, on execution by a suitable computer or processor, the computer or processor is caused to perform the method described herein. The computer readable medium may be, for example, any entity or device capable of carrying the computer program product. For example, the computer readable medium may include a data storage, such as a ROM (such as a CD-ROM or a semiconductor ROM) or a magnetic recording medium (such as a hard disk). Furthermore, the computer readable medium may be a transmissible carrier, such as an electric or optical signal, which may be conveyed via electric or optical cable or by radio or other means. When the computer program product is embodied in such a signal, the computer readable medium may be constituted by such a cable or other device or means. Alternatively, the computer readable medium may be an integrated circuit in which the computer program product is embedded, the integrated circuit being adapted to perform, or used in the performance of, the method described herein.

There is thus provided herein an apparatus, a method and a computer program product that address the limitations associated with the existing techniques.

## Claims

1. An apparatus (12) for controlling a wearable cuff (14) for use in measuring blood pressure, wherein the wearable cuff (14) is inflatable to pressurize a measurement site of a subject (18) and the apparatus (12) is configured to:

   initiate the inflation of the wearable cuff (14) at a first inflation speed; and
   change the first inflation speed during inflation of the wearable cuff (14), **characterized in that** the first inflation speed is changed at a rate that is set to a maximum value by a limiter,
   wherein the maximum value is dependent on
   a pulse rate acquired from the subject (18) during inflation of the wearable cuff (14) at the first inflation speed; and/or
   an amplitude of a signal indicative of pressure oscillations detected in the wearable cuff (14) during inflation of the wearable cuff (14) at the first inflation speed.

2. An apparatus (12) as claimed in claim 1, wherein the apparatus (12) is configured to change the first inflation speed to a second inflation speed during inflation of the wearable cuff (14).

3. An apparatus (12) as claimed in claim 1 or 2, wherein the apparatus (12) is configured to change the first inflation speed by:

increasing the first inflation speed during inflation of the wearable cuff (14); or
decreasing the first inflation speed during inflation of the wearable cuff (14).

4. An apparatus (12) as claimed in any one of the preceding claims, wherein the first inflation speed is changed at a rate that is the maximum value or a rate that is less than the maximum value.

5. An apparatus (12) as claimed in any one of the preceding claims, wherein the first inflation speed is changed at a rate that is variable or a rate that is constant.

6. An apparatus (12) as claimed in any one of the preceding claims, wherein the maximum value is equal to or less than 16 mmHg/s$^2$.

7. An apparatus (12) as claimed in any one of the preceding claims, wherein the apparatus (12) is configured to:
change the first inflation speed based on a pulse rate acquired from the subject (18) during inflation of the wearable cuff (14) at the first inflation speed.

8. An apparatus (12) as claimed in any one of the preceding claims, wherein the apparatus (12) is further configured to:

acquire a signal indicative of pressure oscillations detected in the wearable cuff (14) during inflation of the wearable cuff (14); and
determine a blood pressure value for the subject (18) based on the acquired signal.

9. An apparatus (12) as claimed in claim 8, wherein the acquired signal is in a frequency range from 0.5 Hz to 5 Hz.

10. A system (10) for use in measuring blood pressure, the system (10) comprising:

the apparatus (12) as claimed in any one of the preceding claims; and
the wearable cuff (14) that is inflatable to pressurize the measurement site of the subject (18).

11. A method (300) of controlling a wearable cuff (14) for use in measuring blood pressure, wherein the wearable cuff (14) is inflatable to pressurize a measurement site of a subject, the method (300) comprising:

initiating (302) the inflation of the wearable cuff (14) at a first inflation speed; and
changing (304) the first inflation speed during inflation of the wearable cuff (14), wherein the first inflation speed is changed at a rate that is set to a maximum value by a limiter,
wherein the maximum value is dependent on
a pulse rate acquired from the subject (18) during inflation of the wearable cuff (14) at the first inflation speed; and/or
an amplitude of a signal indicative of pressure oscillations detected in the wearable cuff (14) during inflation of the wearable cuff (14) at the first inflation speed.

12. A computer program product comprising a computer readable medium, the computer readable medium having computer readable code embodied therein, the computer readable code being configured such that, on execution by the apparatus as claimed in claim 1, the apparatus is caused to perform the method as claimed in claim 11.

**Patentansprüche**

1. Ein Gerät (12) zum Steuern einer tragbaren Manschette (14) für die Blutdruckmessung, wobei die tragbare Manschette (14) aufgeblasen werden kann, um die Messstelle am Patienten (18) unter Druck zu setzen, und wobei das Gerät (12) folgende Schritte durchführt:

Einleiten das Aufblasen der tragbaren Manschette (14) mit einer ersten Aufblasgeschwindigkeit; und
Ändern der ersten Aufblasgeschwindigkeit während des Aufblasens der tragbaren Manschette (14),
**dadurch gekennzeichnet, dass** die erste Aufblasgeschwindigkeit mit einer Rate geändert wird, die mithilfe eines Begrenzers auf einen Maximalwert eingestellt ist, wobei der Maximalwert abhängig ist von der Herzfrequenz, die beim Aufblasen der tragbaren Manschette (14) mit der ersten Aufblasgeschwindigkeit vom Patienten (18) abgerufen wird; und/oder von der Amplitude eines Signals, das die Druckschwankungen angibt, die in der tragbaren Manschette (14) beim Aufblasen der tragbaren Manschette (14) mit der ersten Aufblasgeschwindigkeit erfasst werden.

2. Ein Gerät (12) gemäß Anspruch 1, wobei das Gerät (12) beim Aufblasen der tragbaren Manschette (14) die erste Aufblasgeschwindigkeit in eine zweite Aufblasgeschwindigkeitändert.

3. Ein Gerät (12) gemäß Anspruch 1 oder 2, wobei das Gerät (12) die erste Aufblasgeschwindigkeit mithilfe folgender Schritte ändert:

Erhöhen der ersten Aufblasgeschwindigkeit während des Aufblasens der tragbaren Manschette (14);
oder Verringern der ersten Aufblasgeschwindigkeit während des Aufblasens der tragbaren Manschette (14).

4. Ein Gerät (12) gemäß einem der vorherigen Ansprüche, wobei die erste Aufblasgeschwindigkeit mit einer Rate geändert wird, die dem Maximalwert entspricht, oder die kleiner ist als der Maximalwert.

5. Ein Gerät (12) gemäß einem der vorherigen Ansprüche, wobei die erste Aufblasgeschwindigkeit mit einer variablen oder einer konstanten Rate geändert wird.

6. Ein Gerät (12) gemäß einem der vorherigen Ansprüche, wobei der Maximalwert höchstens 16 mmHg/s$^2$ entspricht.

7. Ein Gerät (12) gemäß einem der vorherigen Ansprüche, wobei das Gerät (12) folgenden Schritt durchführt:
abhängig von der beim Aufblasen der tragbaren Manschette (14) mit der ersten Aufblasgeschwindigkeit vom Patienten (18) abgerufenen Herzfrequenz Ändern der ersten Aufblasgeschwindigkeit.

8. Ein Gerät (12) gemäß einem der vorherigen Ansprüche, wobei das Gerät (12) zudem folgende Schritte durchführt:

Abrufen eines Signals, das die Druckschwankungen angibt, die in der tragbaren Manschette (14) beim Aufblasen der tragbaren Manschette (14) erfasst werden; und
Ermitteln des Blutdrucks des Patienten (18) anhand des erfassten Signals.

9. Ein Gerät (12) gemäß Anspruch 8, wobei das erfasste Signal in einem Frequenzbereich von 0,5 Hz bis 5 Hz liegt.

10. Ein System (10) für die Blutdruckmessung, wobei das System (10) Folgendes umfasst:

das Gerät (12) gemäß einem der vorherigen Ansprüche; und
die tragbare Manschette (14), die aufgeblasen werden kann, um Druck auf die Messstelle am Patienten (18) auszuüben.

11. Eine Methode (300) zum Steuern einer tragbaren Manschette (14) für die Blutdruckmessung, wobei die tragbare Manschette (14) aufgeblasen werden kann, um Druck auf die Messstelle am Patienten auszuüben, wobei die Methode (300) folgende Schritte umfasst:

Einleiten (302) das Aufblasen der tragbaren Manschette (14) mit einer ersten Aufblasgeschwindigkeit; und
Ändern (304) der ersten Aufblasgeschwindigkeit während des Aufblasens der tragbaren Manschette (14),
wobei die erste Aufblasgeschwindigkeit mit einer Rate geändert wird, die mithilfe eines Begrenzers auf einen Maximalwert eingestellt ist,
wobei der Maximalwert abhängig ist von der Herzfrequenz, die beim Aufblasen der tragbaren Manschette (14) mit der ersten Aufblasgeschwindigkeit vom Patienten (18) abgerufen wird; und/oder
von der Amplitude eines Signals, das die Druckschwankungen angibt, die in der tragbaren Manschette (14) beim Aufblasen der tragbaren Manschette (14) mit der ersten Aufblasgeschwindigkeit erfasst werden.

12. Ein Computerprogrammprodukt, das ein von einem Computer lesbares Medium mit von einem Computer lesbaren Code umfasst, wobei der von einem Computer lesbare Code beim Ausführen auf dem Gerät gemäß Anspruch 1 dieses dazu veranlasst, die Methode gemäß Anspruch 11 auszuführen.

**Revendications**

1. Appareil (12) de commande un brassard (14) portable destiné à être utilisé pour mesurer la pression artérielle, dans lequel le brassard (14) portable est gonflable pour pressuriser un site de mesure d'un sujet (18) et l'appareil (12) est conçu :

   pour commencer le gonflage du brassard (14) portable à une première vitesse de gonflage ; et
   pour modifier la première vitesse de gonflage lors du gonflage du brassard (14) portable,
   **caractérisé en ce que** la première vitesse de gonflage est modifiée à une vitesse,
   laquelle est fixée à une valeur maximale par un limiteur,
   dans lequel la valeur maximale dépend d'une fréquence de pouls acquise du sujet (18) lors du gonflage du brassard (14) portable à la première vitesse de gonflage ; et/ou
   une amplitude d'un signal indiquant des oscillations de pression détectées dans le brassard (14) portable lors du gonflage du brassard (14) portable à la première vitesse de gonflage.

2. Appareil (12) selon la revendication 1, dans lequel l'appareil (12) est conçu pour changer la première vitesse de gonflage en une seconde vitesse de gonflage lors du gonflage du brassard (14) portable.

3. Appareil (12) selon la revendication 1 ou 2, dans lequel l'appareil (12) est conçu pour modifier la première vitesse de gonflage :

   par augmentation de la première vitesse de gonflage lors du gonflage du brassard (14) portable ;
   ou
   par diminution de la première vitesse de gonflage lors du gonflage du brassard (14) portable.

4. Appareil (12) selon l'une quelconque des revendications précédentes, dans lequel la première vitesse de gonflage est modifiée à une vitesse, laquelle est la valeur maximale ou une vitesse, laquelle est inférieure à la valeur maximale.

5. Appareil (12) selon l'une quelconque des revendications précédentes, dans lequel la première vitesse de gonflage est modifiée à une vitesse, laquelle est variable ou à une vitesse, laquelle est constante.

6. Appareil (12) selon l'une quelconque des revendications précédentes, dans laquelle la valeur maximale est égale ou inférieure à 16 mmHg/s$^2$.

7. Appareil (12) selon l'une quelconque des revendications précédentes, dans lequel l'appareil (12) est conçu :
   pour changer la première vitesse de gonflage en fonction d'un pouls acquis du sujet (18) lors du gonflage du brassard (14) portable à la première vitesse de gonflage.

8. Appareil (12) selon l'une quelconque des revendications précédentes, dans lequel l'appareil (12) est en outre conçu :

   pour acquérir un signal indiquant les oscillations de pression détectées dans le brassard (14) portable lors du gonflage du brassard (14) portable ; et
   pour déterminer une valeur de pression artérielle pour le sujet (18) en fonction du signal acquis.

9. Appareil (12) selon la revendication 8, dans lequel le signal acquis est dans une plage de fréquences comprise entre 0,5 Hz et 5 Hz.

10. Système (10) destiné à être utilisé pour mesurer la pression artérielle, le système (10) comprenant :

    l'appareil (12) selon l'une quelconque des revendications précédentes ; et
    le brassard (14) portable, lequel est gonflable pour pressuriser le site de mesure du sujet (18).

11. Procédé (300) de commande d'un brassard (14) portable destiné à être utilisé pour mesurer la pression artérielle, dans lequel le brassard (14) portable est gonflable pour pressuriser un site de mesure d'un sujet, le procédé (300) comprenant :

le commencement (302) du gonflage du brassard (14) portable à une première vitesse de gonflage ; et
le changement (304) de la première vitesse de gonflage lors du gonflage du brassard (14) portable,
dans lequel la valeur maximale dépend d'une fréquence de pouls acquise du sujet (18) lors du gonflage du brassard (14) portable à la première vitesse de gonflage ; et/ou
une amplitude d'un signal indiquant les oscillations de pression détectées dans le brassard (14) portable lors du gonflage du brassard (14) portable à la première vitesse de gonflage.

12. Produit de programme informatique comprenant un support lisible par ordinateur, le support lisible par ordinateur comportant un code lisible par ordinateur incorporé dans celui-ci, le code lisible par ordinateur étant conçu de telle sorte que, lors de l'exécution par l'appareil selon la revendication 1, l'appareil est amené à mettre en œuvre le procédé selon la revendication 11.

Fig. 1

Fig. 2

300

302

304

Fig. 3

Fig. 4

Fig. 6

Fig. 5

Fig. 9

Fig. 8

Fig. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9017264 B